# EUROPEAN PATENT APPLICATION

(11) **EP 2 213 631 A2**
(43) Date of publication of application: **04.08.2010**
(21) Application number: 08805336.8
(22) Date of filing: 17.07.2008
(51) Int. Cl.: C02F 11/04, C02F 11/18, B01J 19/24, C12P 5/02, C02F 11/00, B01J 8/18

(54) **REACTOR AND ENERGY INTEGRATION SYSTEM FOR CONTINUOUS THERMAL OR THERMOCHEMICAL HYDROLYSIS OF ORGANIC MATERIAL**

(30) Priority: 17.07.2007 ES 200701993
(71) Applicant: SOCIEDAD GENERAL DE AGUAS DE BARCELONA, S.A., 08018 BARCELONA (ES)
(72) Inventor: FERNANDEZ-POLANCO FERNANDEZ DE MOREDA, Fernando, E-47002 Valladolid (ES); PEREZ ELVIRA, Sara l., E-47011 Valladolid (ES); ROUGE ROUGE, Philippe, E-08018 Barcelona (ES); GONZALEZ CALVO, Rafael, E-47011 Valladolid (ES); CANTERO GUTIERREZ, Pilar, E-47002 Valladolid (ES); FERNADEZ-POLANCO IÑIGUEZ DE LA TORRE, Maria, E-47002 Valladolid (ES); RODRIGUEZ GUTIERREZ, Pilar, E-08018 Barcelona (ES); PANIZO CASTAÑO, Luz, E-08018 Barcelona (ES); VELAZQUEZ YUSTE, Roberto, E-47011 Valladolid (ES)
(74) Representative: Sugrañes Patentes y Marcas
(86) International application number: PCT/ES2008/000507
(87) International publication number: WO 2009/010622

(57) **Abstract**

When particulate organic matter is to be subjected to anaerobic digestion, the biodegradability thereof can be facilitated by means of pretreatment of thermal hydrolysis which increases the quantity of biogas produced by more than 50% and reduces the mass of final residue by half. Compared to other processes, the proposed thermal or thermochemical hydrolysis reactor is **characterized in that** it operates continuously at temperatures of up to 190°C and pressures of 12 bar. The biogas produced can be combusted and can produce green electric energy. The hydrolysis reactor is heated by direct injection of steam which can be obtained from the gaseous streams produced in electricity generation and by means of an energy integration system and the hot streams can be exploited thereby making the system energy self-sufficient.

## Description

### "REACTOR AND ENERGY INTEGRATION SYSTEM FOR CONTINUOUS

### THERMAL OR THERMOCHEMICAL HYDROLYSIS OF ORGANIC MATTER"

### Object of the Invention

Anaerobic degradation and consequent biogas production from particulate organic matter is limited by the step of hydrolysis. The invention patent object of the present specification relates to a new treatment process in which, to increase the anaerobic biodegradability, the particulate organic matter is subjected to a thermal hydrolysis process in a reactor designed for that purpose. Said reactor operates continuously, compared to commercial technologies which operate batchwise. Furthermore, in order to make the process energy self-sufficient, an energy integration system with which both the outlet hot streams of the thermal hydrolysis reactor and the exhaust gases and hot water generated during the step of electric energy generation are exploited, is proposed.

The invention can be considered integrated in the field of the art dedicated to the study and optimization of exploitation processes for organic waste, transforming it into others useful for the production of green energies.

### Background of the Invention

In many industrial processes and in particular in the field of waste water and solid waste treatment, streams rich in organic matter are generated. One of the most commonly used processes to recover this waste is to subject it to a anaerobic digestion or biomethanation process. This biological process, developed in the absence of oxygen, transforms the organic matter into biogas, rich in methane, which can be used for electric and thermal energy generation.

According to the scheme of the anaerobic digestion process universally accepted in the case of particulate organic matter, the step which controls the process is the prior hydrolysis or liquefaction, which transforms this particulate matter into soluble compounds accessible for the bacterial metabolism. There are different technologies capable of improving the step of hydrolysis. One of them is thermal hydrolysis. The basis of the process is to subject the particulate matter to a sufficiently high pressure and temperature and thus achieve the solubilization of the solids.

By reviewing the background it can be indicated that different commercial systems perform batch thermal hydrolysis processes. The Cambi process operates in batches and with direct injection of heating steam, seeking as an objective to improve the anaerobic biodegradability of the solids, increasing biogas production. French patent FR 2820735 filed by Vivendi Water Systems in 2001 sponsors the use of two reactors which operate batchwise and in parallel. The batchwise operation forces working in batches, using systems with more complex operation sequences and using reactors with a larger volume.

In relation to continuous operation, the Portheous process applied to previously anaerobically digested sludge, uses reactors with direct injection of steam, seeking to improve the dewaterability characteristics of the sludge. Some patents have proposed systems which operate continuously. U.S. Pat. No. 5593591 of 1997 claims a system in which a sludge pumped under pressure is heated in the conduit itself, before being decompressed by means of a nozzle and entering a flash chamber. The objective of the treatment is to produce a hydrolyzed sludge with good flow characteristics (free flowing solids). U.S. Pat. No. 5785852 of 1998 claims a process and equipment for biological sludge treatment to improve the secondary anaerobic digestion. The multi-step process combines steps of heating, explosive decompression and application of shear forces. A "hydroheater" is the system of mixing the steam and the sludge which is suggested as the most effective. The heated and pressurized sludge is transferred to a flash chamber which operates at atmospheric pressure. The partially disrupted sludge is taken to a new step in which it is subjected to shear forces to complete the cell disruption process. U.S. Pat. No. 2004/0168990 claims a method and device for continuous hydrolysis. The sludge is preheated to approximately 100°C in a heat exchanger and is subsequently mixed, in a undetermined device, with steam of 1 to 4 bar and led to a preheating tank. A pump drives this hot liquid, which in a device external to the hydrolysis reactor receives an injection of steam and penetrates the hydrolysis reactor. The sludge/steam mixture is decompressed to 1 - 4 bar through a nozzle causing an instantaneous pressure drop. The hydrolyzed and heated sludge is pumped from the depressurizing chamber to the first exchanger. The device consists of three pumps, two external devices for mixing steam and sludge. The sole energy recovery systems are expressed in: I) the exchanger preheating the stream of fresh sludge using the hydrolyzed sludge coming out of the decompression chamber as a hot fluid, II) condensation of the steam coming out of decompression chamber in the device prior to the so-called preheating chamber.

The essential features of the proposed invention which make it different from and superior to the inventions presented up until now as it allows minimizing some of their practical limitations, are:
I) The system allows the continuous operation, being clearly different from the commercial Cambi and Vivendi processes
II) It does not need mechanical steps after the step of thermal hydrolysis, as in U.S. Pat. No. 5785852.
III) it does not use external devices for mixing steam like U.S. Pat. No. 2004/0168990.
IV) It does not need pumps operating in high temperature conditions like U.S. Pat. No. 2004/0168990.
V) A suitable mixture is achieved and the temperature gradients are minimized by means of direct injection of steam.
VI) The proposed energy integration allows the excess of biogas produced during hydrolysis to be recovered in the form of green electric energy.

According to the background set forth, the invention is focused in the type and the form of operation of the hydrolysis reactor, as well as in the total exploitation of the energies remaining in the hot streams generated, with the integration of which the energy self-sufficiency of the overall process is achieved.

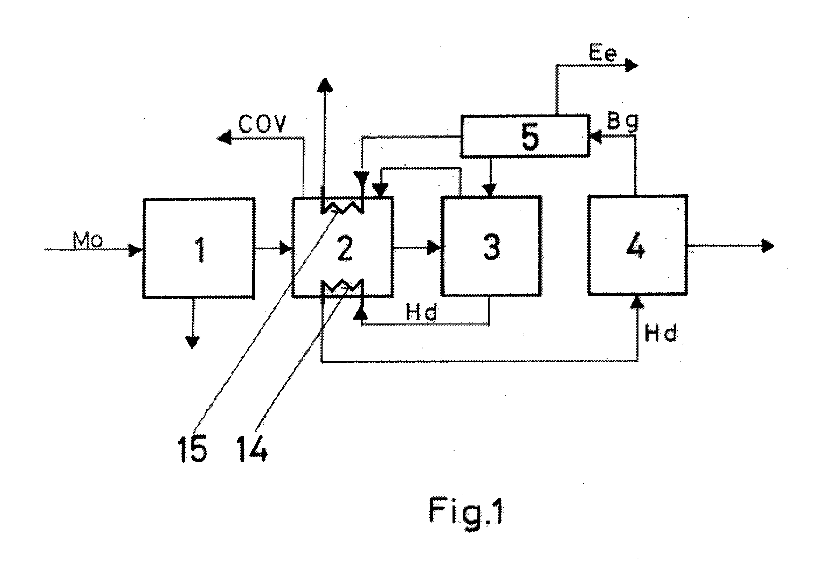

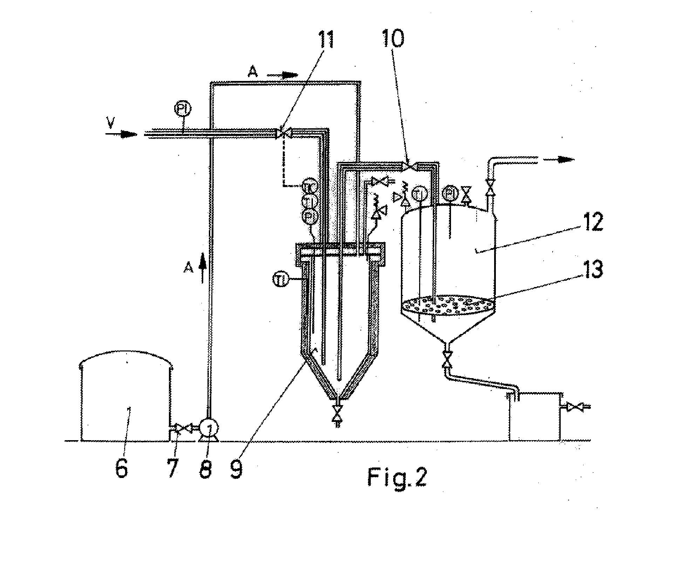

### Brief Description of the Drawing

To aid in better understanding the invention which is described, the attached drawings will be used, which will be referred to by means of the numbers and letters indicated therein.

Figure (1) depicts the block diagram of the hydrolysis process and of the energy integration system; Figure (2) depicts a scheme of the proposed hydrolysis process.

### Disclosure of the Invention

Although slight variants can exist depending on the type of organic matter which is to be hydrolyzed, the general scheme of the process is that appearing in the block diagram of Figure (1). The functions depicted by the different blocks are:
1) Step of concentration of the organic matter (Mo).
   In order for the particulate organic matter to be treated to achieve the operating conditions of the hydrolysis reactor and for its heating to be carried out by means of the exploitation of hot streams, it is dewatered until reaching a suitable concentration level; the preferred process to achieve it is that of centrifugation, although systems for thickening by flotation, sedimentation or filtration in their different variants can also be used; the percentage of solids at the exit of the step of concentration can vary between 1 and 30% total solids.
2) Step of recovery of the thermal energy remaining in the outlet hot streams coming from the depressurizing chamber (step 3) and from the electric energy generation system (step 5).
3) Step of hydrolysis.
4) Step of anaerobic digestion of the hydrolysate (Hd) for biogas (Bg) production.
5) Step of electric energy (Ee) generation by means of a CHP (combined heat and power) assembly from the biogas (Bg) produced during the anaerobic digestion.

A system particularly suitable to carry out the step of hydrolysis (step 2) is presented in claims 1 to 4.

### Detailed Description of an Embodiment of the invention

To explain in detail the operation of the proposed system, from the starting point is biological sludge, with a typical concentration of 3 - 4 g TSS/L (Total Suspended Solid/L), coming from a conventional active sludge process.

After dewatering in step (1) and with an outlet concentration which depending on its composition will vary between 5 and 20%, the semi-solid phase (sludge) passes to step (2) of energy recovery in which it is thermally conditioned by direct injection of steam or by means of heat exchangers which, as will be seen below, receive the streams of hot fluids coming from the step of hydrolysis (3) and from the CHP (combined heat and power) assembly of biogas combustion for the electric energy generation of step (5). After being thermally conditioned, the sludge passes to a storage tank (6) in which suitable quantities of acid or base chemical reagents allowing the subsequent performance of the thermochemical hydrolysis process can be introduced. To assure the absence of gaseous emissions and of odours, the non-condensable gases and the volatile organic compounds (VOCs) accompanying them will be led to a step of auxiliary treatment.

From the tank (6), the thermally and chemically conditioned sludge is pressurized to approximately 10 bar by means of a variable flow pump (8) which continuously introduces it in the hydrolysis reactor (9) .The pump (8) will be suitable for the moving of sludges, positive cavity displacement or membrane pumps being recommendable. The hydrolysis reactor (9) has a cylindrical body and a frustoconical bottom to prevent the formation of deposits; the steam coming from a generator (which is not depicted), enters the reactor through a conduit (V) reaching its frustoconical bottom and condensates heating the mass of sludge to be hydrolyzed which, in turn, enters through the upper part of the reactor through the conduit (A); the residence time of the sludge in the reactor can vary between 5 and 50 minutes; the utile volume of the reactor is determined by means of level, pressure or temperature sensors existing therein, which act on the variable flow feed pump (8) or on the automatic decompression valve (10); the reactor is equipped with temperature (TI) and pressure (PI) sensors which act, by means of a controller (TIC), on the automatic valve (11) placed in the steam inlet pipe (V); the control performed on the valve (11) will maintain the pressure and the temperature in the setpoint values of the operation. It is provided that a portion of steam can be introduced in a timed manner in the reactor (9) so that the sludge under a hydrolyzation process is maintained with the suitable temperature and degree of homogenization, preventing temperature gradients from occurring. The reactor (9) is equipped with the corresponding venting elements, relief and discharge valves and cleaning system.

According to the performed experiments, work is carried out with temperatures comprised between 120°C and 200°C and pressures varying between 4 and 12 bar.

From the hydrolysis reactor (9), the hydrolyzed sludge (Hd) is led to the depressurizing or flash chamber (12) which has a cylindrical body, frustoconical bottom and torispherical upper lid; the passage of the sludge to this chamber is performed in a controlled manner by means of the decompression valve (10) through a tubular conduit which, starting from the lower part of the frustoconical bottom of the reactor (9), with which it remains coaxial, reaches the frustoconical bottom of the tank (12); the valve (10) can be automatically manoeuvred by timing or commanded by the level sensors, (not depicted) existing in the hydrolysis reactor (9); this valve (10) is located very close to the chamber (12) so that its opening causes in the reactor (9) a sudden pressure drop which collaborates in the establishment in the tank (12) of an abrupt steam expansion mechanism (steam explosion) which favours the overall efficiency of the hydrolysis process. To attenuate the effect of this expansion, the tank (12) is provided with a perforated deflection plate (13), normal to its axis, internally dividing it into two areas; the tank (12) is also equipped with temperature sensors (TI), pressure sensors (PI) and with the corresponding venting elements, relief and discharge valves and cleaning system.

The stream of sludge which, coming from the reactor (9), reaches the depressurizing chamber (12) through the automatic valve (10) is split, according to the reactor and chamber pressures and temperatures, into a vapour phase and a liquid phase which are led to step (2) of energy recovery; the hydrolysate (Hd), the liquid phase, with excellent flow characteristics, after crossing the heat exchanger (14) of step (2) is led to step (4) of anaerobic digestion. By maintaining a suitable pressure in the depressurizing chamber (12) which compensates the pressure losses, it will be not necessary to pump the liquid phase.

In step (4), the hydrolysate, alone or mixed with another non-hydrolyzed sludge is digested in an anaerobic process; taking into account the temperature of the hydrolysate the anaerobic digestion can be performed both in a mesophilic and thermophilic manner. In typical hydrolysis conditions, i.e., residence time of 30 minutes, pressure of 7 bar, temperature of 170°C, biogas (Bg) production is between 40 and 60% greater than that obtained when the sludge has not been subjected to the thermal hydrolysis process.

Likewise and taking into account the exceptional conditions of dewaterability of the hydrolysate, its concentrated can be carried out, a liquid fraction and another pasty fraction being obtained. Each of the fractions is taken to a different anaerobic digester, to thus independently optimize the mixing conditions and the residence time of each fraction.

The biogas (Bg) produced during the digestion process of the hydrolyzed sludge is taken to step (5), in which a commercial CHP (combined heat and power) assembly uses it to produce "green" electric energy (Ee) and hot residual streams of gases or water which are led to step (2), to recover the remaining thermal energy thereof by means of the heat exchanger (15).

With the proposed arrangement and operating form it is achieved that the biogas production increases by more than 40% and that it can be used, in its entirety, in the electric energy production.

Taking into account that most of the energy required for the hydrolysis process is heat energy which is recovered as a result of the proposed energy integration process, the system is globally self-sufficient.

The invention is not strictly limited to the described embodiment but rather it comprises any other embodiment variant.

The semi-finished materials, commercial parts and integrated assemblies as well as the manoeuvre, measurement and control equipment the use of which is provided in the invention, are those currently used in the facilities where precision chemical processes are performed.

## Claims

1. A reactor and energy integration system for continuous thermal or thermochemical hydrolysis of organic material **characterized by** the use of a chemical reactor associated with a depressurizing chamber; the reactor has a cylindrical body, flat lid and frustoconical bottom; the depressurizing chamber has a cylindrical body, torispherical lid, conical bottom and a perforated circular plate normal to its axis, internally dividing it into two areas; the reactor and chamber are communicated with one another by means of a tubular conduit which penetrates through the respective lids, reaching the lower part of its frustoconical bottom at one end, coaxially with the reactor, and the frustoconical bottom of the depressurizing chamber at the other end; a valve automatically manoeuvrable by timing or by command of the level sensors existing in the reactor is intercalated in this tubular conduit; a conduit for injection of steam, coming from an external generator the features of which are not claimed, enters through the lid of the reactor to its frustoconical bottom, which conduit is provided with a valve automatically manoeuvrable by timing or by command of the pressure and temperature sensors with which the reactor is provided.

2. The reactor and energy integration system for continuous thermal or thermochemical hydrolysis of organic material according to claim 1, **characterized by** the use of a storage tank communicated with the reactor by means of a tubular conduit which slightly penetrates the latter through its lid; a valve at the outlet of the storage tank and a pump suitable for driving sludges continuously and in a variable flow automatically regulated by command of the level, pressure and temperature sensors existing in the reactor are successively intercalated in this conduit.

3. The reactor and energy integration system for continuous thermal or thermochemical hydrolysis of organic material according to the previous claims, **characterized by** using a commercial CHP (combined heat and power) assembly, suitable for combusting biogas, the features of which are not claimed.

4. The reactor and energy integration system for continuous thermal or thermochemical hydrolysis of organic material according to the previous claims, **characterized by** using heat exchanger and direct steam injection devices, communicated by means of tubular conduits with the depressurizing chamber and with a commercial CHP (combined heat and power) assembly.
